# EUROPEAN PATENT APPLICATION

(11) **EP 1 974 710 A1**
(43) Date of publication of application: **01.10.2008**
(21) Application number: 07006222.9
(22) Date of filing: 26.03.2007
(51) Int. Cl.: A61H 7/00, A61H 23/02

(54) **Finger pressing massage glove**

(71) Applicant: Tsai, Shen-Hai, Taichung County 428 (TW)
(72) Inventor: Tsai, Shen-Hai, Taichung County 428 (TW)
(74) Representative: Becker Kurig Straus

(57) **Abstract**

The present invention is a fingertip massaging glove; particularly, a glove massager to facilitate a user in operation according to his preference. The glove massager includes a glove fit out one's hand for vibration, a vibration device enclosed inside of the glove includes a panel with an interface displayed on a hand back of the glove, a number of eccentric vibrators connected to the panel by leads. A set of batteries are disposed inside the panel to supplement power to the glove. When the vibrators are powered to vibrate, affected part of a user can be relieved pain or sore. Furthermore, a second side of the glove includes palm, fingertips portions embossed with grip dots has a depth deeper than a normal breadth of the glove.

## Description

### BACKGROUND OF THE INVENTION

### (a) Field of the Invention

The present invention is related to a finger pressing massage glove, which is portable, easy to preserve, and operated according to user's preference.

### (b) Description of the Prior Art

Gloved hand massaging is manipulated by user's hand; persons skilled in the art have provided kinds of glove massager. For instance, US 6,748,604 entitled "Glove massager" to Duboff et al. on 06/15/04 disclosed a glove which includes massage elements within each of the fingertips of the glove. Each massage element includes a housing and a motor therein for vibrating the housing. Or US 6,203,509 entitled "Fingertip massager" to Gary Duboff on 03/20/01, a plurality of panels attachable to and detachable from the housing of the massager for vibration with the housing on fingertip. A securing strap is provided for attaching the device to the single finger of a user.

Obviously, fingertip massager mountable to a user's fingertip can be assembled to form a glove massager worn by hand; only the massage elements can be located proximate to the palm. A massage element is not only shaped with a convex (i.e. hemispheric) or concave surface, but a first portion of the housing abuts against topside of the finger, a second portion detachable from the housing one may obtain access to a motor for its servicing or replacement. Maintenance work of separate fingertip massagers assembled on a glove will be hard to be fulfilled. Since there are numerous portions, convex and concave surfaces among fingertips and palm, securing straps or fasteners are required for attaching massagers to fingertips or center of palm, or wrist; otherwise, massagers may be vibrated to go wrong track, or the glove may drop out thereof. Massaging effect is dependent on hemispheric massage element or node activated by motor only, no other options, no more marginal utilities can be expected.

A massager can be embodied by a permanent magnet such as published US 2005137445 entitled "eye massager for receiving magnetic devices" to Chang et al. on 06/23/05. A mask body is formed with two through holes at positions corresponding to those of user's eyes. Each through hole enclosed by a rubber ring is passed through by and fixed with a permanent magnet sealed in a handle by a plug. Magnetic fields generated from the permanent magnet will pass through bottom planes and grooves of the plugs to activate the eye's nerves so as to adjust the function of eyes. But magnets incorporated into a glove massager for vibration may be a time consuming task, because magnets should be identified and arrayed according to their polarities or magnetic force, such as a massager need to loose grip or knuckle, permanent magnets could not produce constant vibrations to the glove. Further, to manipulate such a magnetic glove massager worn by hand, the user must exert more force through his palm, or fingers.

Or embodied as published US 200621961 entitled "Massager with shock absorption, multiple contact surface and visual therapy effects" to Meyer Elizabeth et al. on 09/21/06, basically two or more hemispheric massage nodes are expected from US 200621961 as described in its parent patented case US 7,128,722, entitled "Percussive massager with variable node spacing" to Mordechai et al. on 10/31/06 incorporated into US 200621961. To reduce shock and vibration imparted to hand or wrist, spring and dampener are aligned coaxially at a connection between handle and massage head. The dampener may contain gel, fluid and so on to improve the absorption of shock and vibration. It is assumed that the anti-shock dampener is mainly dependent on the co-axial spring, but such a combination of spring and dampener is further restricted to a linear movement likely applications of Hook's law.

Moreover, use of latex gloves and finger cots in combination with lubricants, vinyl gloves-which are traditionally used to replace tatex-are even worse as far as permeability and damage from non-water based lubricants are concerned. Therefore, a glove embossed with hundreds of "grip dots" distributed over its surface in contact with skin, at least, will be a better choice for users than traditional gloves or cots, such grip dots is incorporated into the glove massager of the invention.

### SUMMARY OF THE INVENTION

Accordingly, the present invention is to provide an eccentric vibration glove massager being portable, easy to preserve, and manipulated according to user's preferences.

Point against aforesaid defects of prior arts, the present invention is to provide a glove massager comprising a glove 10 worn by hand; a plurality of grip dots 11 are distributed over a second side included center of palm and fingertips surfaces of the glove 10; a vibration device 20 enclosed inside of the glove 10 includes a panel 21 displayed on a first side of hand back portion of the glove 10, the panel 21 has an interface 21 h, a number of eccentric vibrators 23 connected to the panel 21 by leads 22, a set of batteries 21 g mounted inside the panel 21 as a power source; the panel 21 is equipped with a first button 21 a with two keys to activate vibrator and grip dots on a thumb portion, an index finger portion respectively, a second button 21 b to activate vibrators and grip dots of the glove, a frequency button 21c with forward and backward function keys for increment or decrement operation frequency, and a power switch 21 d for switching on or off power source.

Glove massager as mentioned above, the eccentric vibrators 23 are located on the fingertips of the glove 10.

Glove massager as mentioned above, the eccentric vibrators 23 are located on a palm portion of the glove 10.

Glove massager as mentioned above, the eccentric vibrators 23 are located on a hand back portion of the glove 10.

Glove massager as mentioned above, the eccentric vibrator 23 is enclosed inside a housing 24, which is shaped as a tube.

Glove massager as mentioned above, the panel has a number of light emitting diodes (LEDs) 21 e for showing response signals.

Glove massager as mentioned above, the set of batteries 21g are direct current (DC) 650-900 milli-Ampere-hour capacity (AAA) batteries or Nickel-Metal Hybride (Ni-Mh) AAA rechargeable batteries.

Glove massager as mentioned above, a transformer converts alternate current (AC) into direct current (DC) to the rechargeable batteries.

### BRIEF DESCRIPTION OF THE DRAWINGS

Fig. 1 shows an exploded view of the glove massager.
Fig. 2 shows a perspective view of the glove massager.
Fig. 2A shows an enlarged elevated view of the panel of Fig. 2.
Fig. 2B shows an enlarged exploded view of the eccentric vibrator of Fig. 2.
Fig. 3 shows a perspective view of the glove massager with the thumb portion pointing up toward affected area.
Fig. 4 shows a side view of the glove massager moved the thumb portion and mitten portion.
Fig. 5A shows a diagrammatic view of the eccentric vibrator in rotary motion in clockwise.
Fig. 5B shows a cross sectional view of the eccentric vibrator of Fig. 5A.
Fig. 6A shows a diagrammatic view of the eccentric vibrator in rotary motion in clockwise following up Fig. 5A.
Fig. 6B shows a cross sectional view of the eccentric vibrator of Fig. 5B.

### DETAILED DESCRIPTION OF THE PREFERRED EMBODIMENTS

The description is described in detail according to the appended drawings hereinafter.

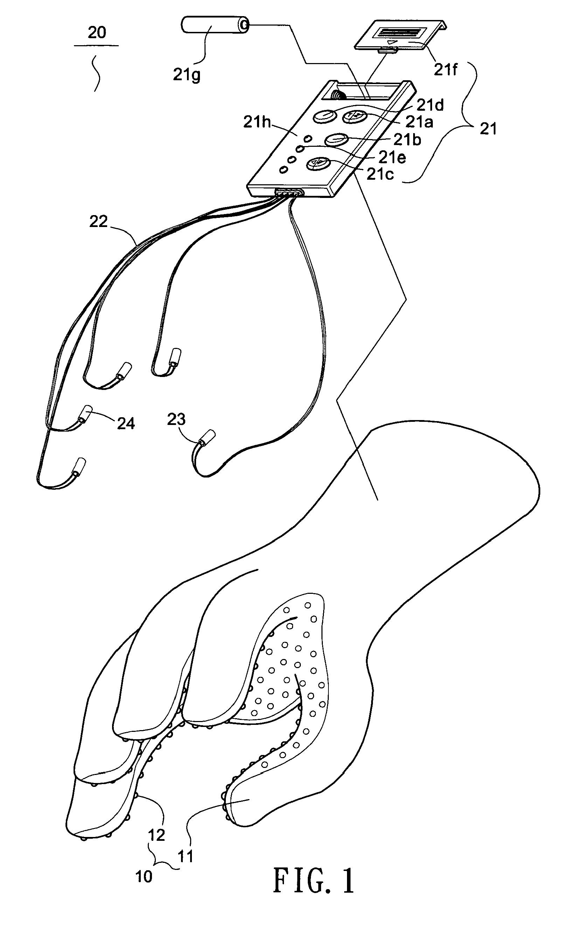

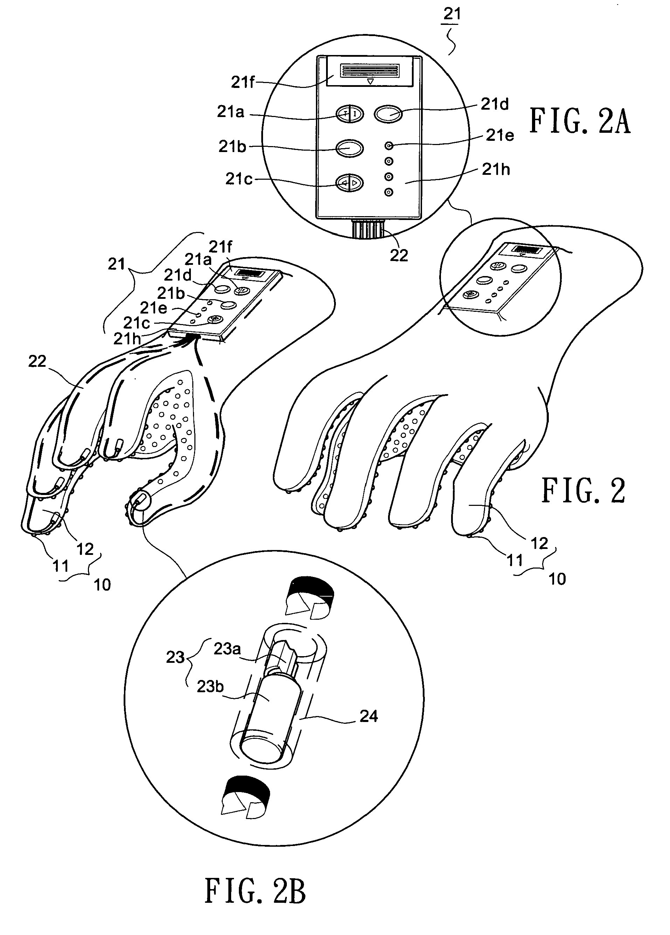

Referring to Figs. 1 and 2, a glove massager 20 equipped with eccentric vibrators 23 is composed of a glove 10, and a vibration device 20 is hidden from view and enclosed inside of the glove 10. The vibration device 20 includes a panel 21 displayed an interface 21 h on a first side (i.e. hand-back portion) of the glove 10. The panel 21 has a set of batteries 21 g to supply power to the glove 10. A number of leads 22 are connected to a number of eccentric vibrators 23 on fingertips, palm, and hand-back portions respectively. In other words, the vibrators 23 are in parallel connection with the panel 21 to be operated subsequently. Such vibrators in parallel connection to the panel are mainly divided into a first dominated portion, a second attached portion. But the glove takes shape of user's hand is obviously divided into thumb and mitten portions with alternative postures-either a pointed or a curved posture-can be practiced for a grip massaging; but the vibrators 23 of fingertips etc., are activated by pressing buttons 21 a or 21 b. A massage grip can be formed by means of the mitten portion in a coordination action with the thumb portion of the glove, or a thumb portion pointing up is directly opposite to an affected part. Or a vibrator of an index fingered portion is activated duly for the patient's use. Or a thumb portion and an index finger portion of the glove can be combined in use. Thereby, a first button 21a of the panel 21 can activate a thumb portion, an index fingered portion, or both according to the user's preference. Other than the mitten portion is in coordination with the thumb portion with an enhanced massage effect, a glove massager put on hand is to be dominated by vibrators of the thumb portion or the index fingered portion to exert pressure to relieve a local aching; but vibrators of a second attached portion, namely part of the mitten portion includes a middle, ring and little fingered portion, are not activated independently but attached to the first dominated portion in use. Vibrators of both the first dominated and the second attached portions of such a glove can be activated by a second button 21 b of the panel 21. But only first dominated portion can be activated by the button 21 a optionally. Vibrators can also be added to a second side (i.e. center of palm portion) of the glove, and a first side (i.e. hand back portion) about the wrist, once the palm portion or hand back portions are added with vibrators, they are broadly defined and included in the mitten portion and controlled by the second button 21 b, all of the vibrators 23 can be activated by pressing the second button 21 b of the panel 21 in one step. A hand-back portion added with vibrator can be suitable for a pet massage in contact with soft fur instead of massaging grip. And a palm portion added with vibrators can also expand the massaging area. To activate hand-back portions vibrators can also be suitable for massaging with a knuckle. Both the palm and hand-back portion vibration can be suitable for massaging affected areas about armpits or calf and behind knee or shoulder about neck by a patient himself.

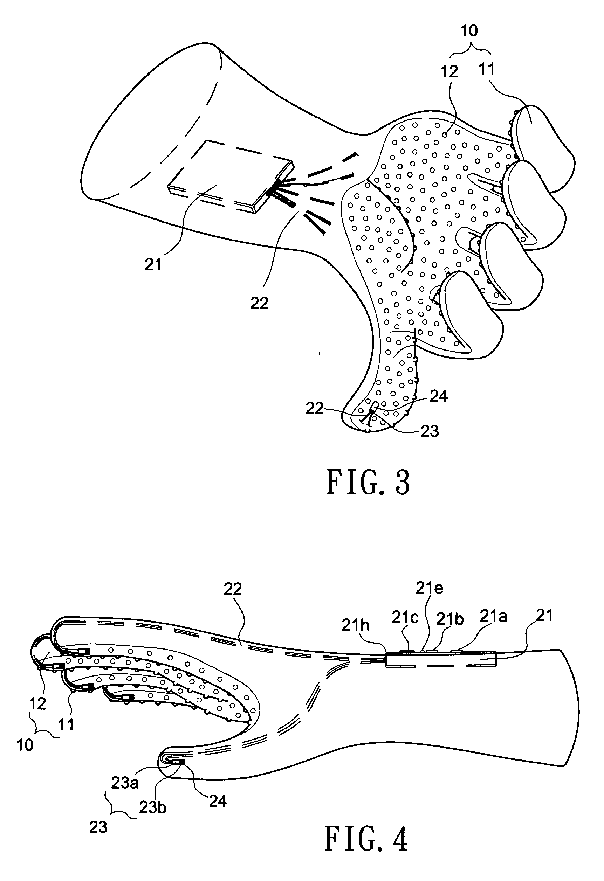

Referring to Fig. 2A, the panel 21 is equipped with buttons 21 a, 21 b, and 21c and power switch 21d. Where the button 21a having T key electrically connected to the thumb portion and I key electrically connected to the index fingered portion, vibrators of the thumb, index fingered portions can be activated immediately by pressing the T, or I key of button 21 a respectively. A user can optionally select the thumb portion, the index fingered portion or both for vibration by pressing T key or I key or both keys of the button 21a at the same time. The glove is made of synthesis resin, rubber, or silicone to form a heavy rubber glove; further, the second side of the glove 10 is distributed with grip dots; therefore, as the mitten portion of the glove 10 takes user's hand shape, at least, can be implemented in a normally curved posture as Fig. 2 depicted or curling around posture as Fig. 3 depicted or pointing up posture as Fig. 4 depicted. The button 21 b is designed to activate not only the first dominated portion, but also the second attached portion. While a second side (i.e. center of palm), or a first side (i.e. back of hand) of the glove 10 can also be equipped with vibrators activated by the same button 21 b for vibration. According to such an arrangement, either the first side (i.e. back of hand) or the second side (i.e. palm portion) for vibration is not separated from the mitten portion and controlled by the second button 21 b. The button 21 c is further provided with a forward and a backward function key indicated that the frequency of vibrators can be adjusted higher or lower than a default frequency by pressing the forward or backward function key, for example, the button 21c may be combined with a sliding variable resistor which permits the speed of a motor and hence the frequency of the vibrations generated by eccentric mass to be controlled, once pressing on the function key comes to a halt, that is a "broken circuit" happened to the button 21c. User can adjust the frequency anew by pressing one of the function keys or both. The power switch 21 d is to switch on or off the power source. All the buttons and switch can be operated according to user's preference. A number of light emitting diodes (LEDs) 21e are responsive to the operations of the buttons and switch; accordingly, statuses of the glove 10 in use can be easily inferred from signals shown by LEDs 21 e.

The glove 10 is made of flexible rubber, silicone, synthesis resin etc., all snugly fit one's hand. A lid 21f is covered over the set of batteries 21g to reduce electricity leakage further facilitate replacement of used batteries with new batteries.

Eccentric vibrators 23 of the invention can be known from use of eccentric masses concurrently rotated about parallel axes in opposite directions. Such vibrators may be activated at the same time, but not vibrate at the same frequency, since vibration frequency can be adjusted separately after activating only one portion such as the thumb portion at an adjusted speedy frequency for a while, but to activate the other portion such as the index fingered portion later at a default frequency unless the user adjust the frequency also. Vibrators 23 are mainly installed to fingertips of the glove 10, or center of palm of the glove 10, further can be installed to hand-back portion of the glove 10. In practice, eccentric vibrators are firstly installed to the fingertips portions, secondly to center of palm portion, thirdly to hand-back portion.

The set of batteries 21g are direct current batteries or rechargeable batteries, or alternating current (AC) converted into direct current (DC) supplements power to the rechargeable batteries. The set of batteries 21 g are direct current (DC) 650-900 milli-Ampere-hour capacity (AAA) batteries or Nickel-Metal Hybride (Ni-Mh) AAA rechargeable batteries.

Referring to Fig. 2B, the eccentric vibrator 23 is composed of an eccentric mass 23a and a motor 23b; furthermore, the eccentric vibrator 23 is enclosed by a tubular housing 24.

Referring to Figs. 3 and 4, the mitten portion 12 of the glove 10 further divided into four compartments can accommodate index finger, mid finger, ring finger and little finger wearing the glove corresponding to the compartments in place. Whereby, a pointed up thumb portion as depicted in Fig. 3 or an index finger portion separated from the mitten portion can be used for vibration with as a relief of local aching. Or a grip of the thumb portion in coordination action with mitten portion of a curved posture for massaging as depicted in Fig. 4 or as the second attached portion combined to first dominated portion for vibration as a massaging grip. As the eccentric vibrators 23 are activated, the grip dots 11 distributed over fingertips, center of palm around vibrators 23 are moved to and fro to exert pressure to the affected area. The grip dots 11 are mainly formed on the fingertips and palm integrally with the glove by injection molding. As a result, the second side of the glove is embossed with hundreds of grip dots to increase a depth of the glove with an evenly distributed convex and concave surface popped to the affected area, so that a breadth of the glove is increased to the second side of the glove 10. Where vibrator with grip dots can be kept for a constant vibration since the grip dots are evenly distributed with equal size, equal spacing; therefore, the vibrator can be operated with efficiency. Due to the grip dots have a certain depth increased to the surface of the glove being in contact with patient's skin, at least, grip dots incorporated into the surface of the glove is one and half times deeper than the glove breadth. Where the embossed grip dots can be kept intermittent gaps between the affected part and the second side of the glove, further air-circulation space could be retained, at least, to limit expansion of diseases such as dermatitis etc., from affected part to others directly. Since the grip dots shaped on one side of the glove can be shaped wavelike (such as sinusoidal waveform) with soft pressure effective in supporting the glove massager in contact with skin, further a massage effect increased to the affected part, and grip dots are easy to clean, even can be immersed through non-water based detergents without deterioration.

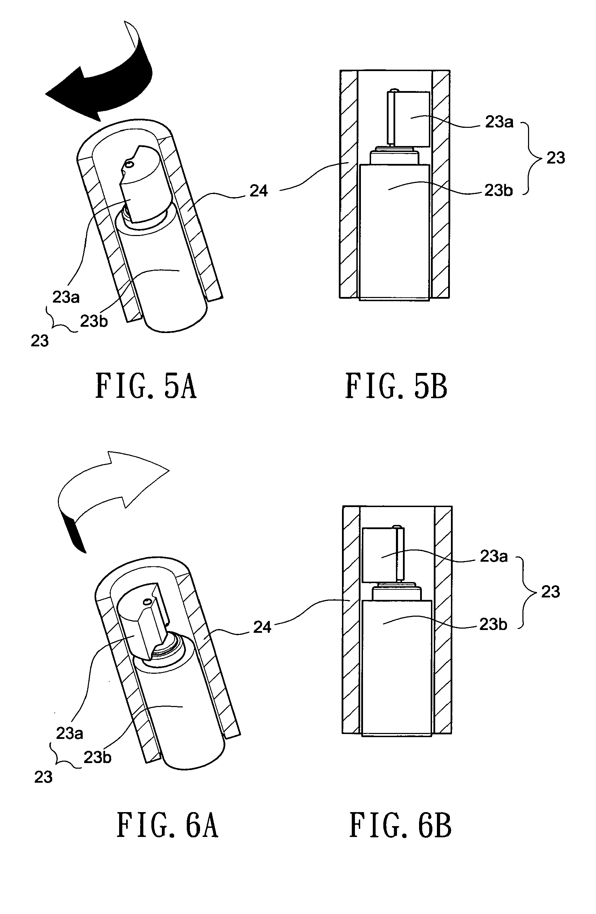

Referring to Figs. 5A, 5B, 6A and 6B, an eccentric vibrator 23 is composed of an eccentric mass 23a and a motor 23b. Owing to the gravity center is not located at a center of the vibrator 23, the eccentric mass 23a of the vibrator 23 may be vibrated to go wrong track, but a housing 24 can locate the eccentric vibrator 23 in place make constant vibration possible, further can be avoided damage from force outside.

In order to further manifest advancement and practicability of the present invention, the advantages thereof are listed below:

Glove massager of the invention is easy to manipulate due to switches 21 a, 21 b, 21 c, and 21 d, can be operated optionally, thereby the massager can relieve a pain or sore affected part. Interface 21 h of the panel 21 is designed to achieve a massage effect according to one's preference. Statuses of massaging can be inferred from response signals shown with a number of LEDs 21e above all ambiguity.

Further, vibration device 20 is hidden from view and enclosed inside of the glove 10 worn by hand. A glove with vibration device put on one's hand from fingertips to the wrist. Since a glove takes user's hand shape is divided into a thumb and a mitten portion, but in use, is substantially divided into a dominated first portion and an attached second portion electrically connected to the activated buttons 21a, 21 b. The mitten portion of the glove takes user's hand shape is further divided into four compartments for fitting in index finger, middle finger, ring finger and little finger with a little curved posture, glove massager worn on hand is neither dropping out nor vibrating to go wrong track. Glove massager made of soft silicone, rubber, or synthesis resin is portable and easy to preserve. Further hundreds of grip dots distributed over the glove massager, on one hand, may increase its depth to protect the user from infected with such as human immunodeficiency virus (HIV); on the other hand, the glove surface embossed with grip dots may be shaped by injection molding in one step. As grip dots are integrally formed on the surface of fingertips, palm, or even hand back, which are easy manipulation and shock absorption.

Fingertips 12 of the glove 10 are separated from each other; hence a thumb portion, or an index fingered portion, or a dominated first portion included both thumb and index finger can exert force that uses trigger point therapy to relieve pain and sore affected areas, fingertips of a mitten portion (i.e. four fingered portion) is divided into four compartments, part of the mitten portion as the second attached portion included middle, ring and little fingered portions etc., can be optionally activated to exert more massage force in addition to the first dominated portion on an affected part by pressing the second button 21 b one and all. User also can put both hands with glove massagers having both, for example, thumb and mitten portions thereon. But, the glove massager can be provided according to one's handedness, or a pair of glove massagers provided to both hands for a user.

A set of batteries 21g are assembled to the panel 21, which is connected to the glove 10 integrally as a whole, a lid 21f is covered over the set of batteries 21g to reduce electricity leakage further facilitate replacement of used batteries with new batteries.

Eccentric vibrator 23 enclosed by a tubular housing 21 may avoid from impacts caused by outer force. The tubular housing 21 also can prevent the eccentric vibrators 23 from abrasion caused by constant friction between glove 10 and vibrators 23. That may cause the eccentric vibrators 23 dropped out of the glove 10.

## Claims

1. A glove massager comprising a glove (10) worn by hand; a vibration device (20) enclosed inside of the glove (10) includes a panel (21) displayed on a hand back portion of a first side of the glove (10), the panel (21) has an interface (21 h), a number of eccentric vibrators (23) connected to the panel (21) by leads (22), a set of batteries (21g) mounted inside the panel as a power source; **characterized in that**: the glove (10) is embossed with a plurality of grip dots (11) distributed over a second side, included center of palm and fingertips surfaces of the glove (10), the panel (21) is equipped with a first button (21 a) having function keys T key, I key to activate vibrator and grip dots of a thumb portion, an index fingered portion respectively; a second button (21 b) to activate vibrators and grip dots of the glove (10); a frequency button (21c) with forward and backward function keys for increment or decrement operation frequency; and a power switch (21 d) to switch on or off power source.

2. The glove massager as claim 1 claimed, the eccentric vibrators (23) are located on the fingertips included thumb portion, index fingered portion, middle fingered portion, ring fingered portion and little fingered portion of the glove (10).

3. The glove massager as claim 1 claimed, the eccentric vibrators (23) are located on a palm portion of the glove (10).

4. The glove massager as claim 1 claimed, the eccentric vibrators (23) are located on a hand back portion of the glove (10).

5. The glove massager as claim 1 claimed, the eccentric vibrator (23) is enclosed inside of a housing (24), which is shaped as a tube.

6. The glove massager as claim 1 claimed, the panel has a number of light emitting diodes (LEDs) (21 e) for showing response signals.

7. A glove massager comprises the set of batteries (21g) are direct current (DC) 650-900 milli-Ampere-hour capacity (AAA) batteries or Nickel-Metal Hybride (Ni-Mh) AAA rechargeable batteries.

8. The glove massager as claim 7 claimed, a transformer converts alternate current (AC) into direct current (DC) to the rechargeable batteries.

9. The glove massager as claim 1 claimed, the glove 10 is a heavy rubber glove made of silicone, synthesis resin, or rubber.

10. The glove massager as claim 1 claimed, the glove 10 is embossed with a plurality of grip dots as heavy rubber glove, at least, palm and fingertips portions with grip dots has a depth one and half times deeper than a breadth of the glove.
